(19) Europäisches Patentamt
European Patent Office
Office européen des brevets

(11) Numéro de publication: **0 130 875**
**B1**

(12) # FASCICULE DE BREVET EUROPÉEN

(45) Date de publication du fascicule du brevet: **22.07.87**

(21) Numéro de dépôt: **84401230.2**

(22) Date de dépôt: **15.06.84**

(51) Int. Cl.⁴: **C 07 C 79/12,** C 07 C 79/35, C 07 C 79/355, C 07 C 79/46, C 07 C 103/22, C 07 C 121/52, C 07 C 125/03

(54) **Procédé d'halogénation-nitration-fluoration de dérivés aromatiques.**

(30) Priorité: **23.06.83 FR 8310371**

(43) Date de publication de la demande: **09.01.85 Bulletin 85/2**

(45) Mention de la délivrance du brevet: **22.07.87 Bulletin 87/30**

(84) Etats contractants désignés: **AT BE CH DE FR GB IT LI LU NL SE**

(56) Documents cité:
**GB-A-955 898**
**GB-A-1 206 389**
**US-A-4 061 688**

**HOUBEN-WEYL: METHODEN DER ORGANISCHEN CHEMIE, vol. V/3, édition 4, 1962, Georg Thieme Verlag, Stuttgart (DE);**

(73) Titulaire: **RHONE- POULENC SPECIALITES CHIMIQUES, "Les Miroirs" 18, Avenue d'Alsace, F-92400 Courbevoie (FR)**

(72) Inventeur: **Desbois, Michel, 526 Chemin du Bois, F-69140 Rillieux (FR)**
Inventeur: **Disdier, Camille, 54 Avenue Cabias, F-69004 Lyon (FR)**

(74) Mandataire: **Cazes, Jean- Marie, RHONE- POULENC INTERSERVICES Service Brevets Chimie 25, quai Paul Doumer, F-92408 Courbevoie Cédex (FR)**

**EP 0 130 875 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention concerne un procédé d'halogénation, de nitration et de fluoration de dérivés aromatiques substitués par au moins un groupe comportant un motif halogénoalkyle. Elle concerne plus particulièrement un procédé d'halogénation et de nitration du noyau aromatique et un procédé de fluoration dudit groupe par échange halogène fluor.

On entend par halogénation la fixation d'au moins un atome de chlore ou de brome sur le noyau aromatique.

On entend par groupe comportant un motif halogénoalkyle, un groupe de formule suivante:

$- A - CX_1X_2Y$

dans laquelle:

A représente une liaison covalente, l'oxygène ou le soufre

$X_1$ et $X_2$ identiques ou différents représentent un halogène

Y correspond à l'hydrogène, à un halogène ou à une chaîne alkyle de 1 à 3 atomes de carbone éventuellement halogénée.

Les halogènes correspondant à $X_1$ $X_2$ et Y sont identiques ou différents mais au moins l'un d'entre eux ne doit pas être le fluor.

Pour plus de clarté on désignera ci-après, l'échange halogène-fluor du groupe comportant un motif halogéno-alkyle par le terme: fluoration-échange.

Il est connu depuis longtemps de procéder dans une première étape à une fluoration-échange de dérivés aromatiques substitués par au moins un groupe comportant un motif halogénoalkyle dans l'acide fluorhydrique. On procède ensuite, dans une deuxième étape indépendante, à une chloration du dérivé aromatique, substitué par au moins un groupe comportant un motif fluoroalkyle, au moyen d'un catalyseur de chloration tel que notamment $FeCl_3$, $BF_3$ (brevet allemand n° 825 397), Pt sur alumine (brevet allemand n° 1 034 609). On effectue enfin dans une troisième étape, la nitration du dérivé aromatique chloré et substitué par un groupe fluoroalkyle au moyen d'un mélange acide nitrique-acide sulfurique tel que décrit dans la demande de brevet européen n° 54464.

Le fait d'opérer en trois étapes présente de nombreux inconvénients et surtout provoque une perte de rendement.

Il a été possible tel que décrit dans Houben Weyl (vol.3 p. 679) de regrouper les deux premières étapes: chloration et fluoration-échange en une seule étape en utilisant un catalyseur tel que le pentachlorure d'antimoine dans l'acide fluorhydrique anhydre, mais il faut ensuite, dans une étape indépendante, procéder à la nitration.

Le procédé précédemment décrit présente l'avantage d'opérer en deux étapes, mais il nécessite la présence de deux catalyseurs $SbCl_5$, pour les deux premières étapes et $H_2SO_4$ pour la nitration; $SbCl_5$ n'étant pas recyclable, cela pose du point de vue technique des problèmes de pollution et du point de vue économique, des problèmes de rentabilité.

La présente invention qui remédie aux inconvénients de l'art antérieur concerne un procédé d'halogénation-nitration-fluoration échange de composés aromatiques substitués par au moins un groupe comportant un motif halogénoalkyle caractérisé en ce que dans une même enceinte réactionnelle, on fait réagir sans traitement intermédiaire successivement ou simultanément ledit composé aromatique avec un halogène et un agent nitrant dans l'acide fluorhydrique liquide. L'acide fluorhydrique joue une double fonction; il sert d'agent de fluoration du groupe substituant le noyau et sert de solvant lors de l'halogénation et de la nitration du noyau aromatique.

On peut selon l'invention faire réagir d'abord l'agent nitrant puis l'halogène ou l'halogène puis l'agent nitrant ou les deux simultanément. On préfère cependant faire réagir d'abord l'halogène puis l'agent nitrant.

Lorsque l'on fait réagir l'agent nitrant puis l'halogène, le dérivé aromatique nitré obtenu étant beaucoup moins réactif, les conditions réactionnelles pour procéder à l'halogénation doivent être plus sévères. D'autre part, lors de la nitration, il y a formation d'eau qui, au cours de l'halogénation, provoque une hydrolyse partielle du groupe halogénoalkyle.

Il est encore possible de procéder simultanément à la nitration et à l'halogénation au sein de l'acide fluorhydrique mais il est certain que dans ce dernier cas, on obtiendra un mélange d'isomères ce qui est rarement un avantage du point de vue industriel.

D'un point de vue pratique, il est donc préférable de procéder d'abord à l'halogénation puis à la nitration.

Le composé aromatique mis en oeuvre dans le cadre de l'invention répond de préférence à la formule (I) suivante:

$Ar-(ACX_1X_2Y)_n$ (I)

dans laquelle:

Ar représente un radical aromatique mono ou polycyclique

A, $X_1$ $X_2$ et Y ont la signification préalablement mentionnée

n est égal à 1 ou 2 et de préférence égal à 1

Ar a de préférence pour formule:

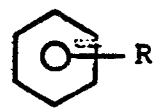

dans laquelle R représente un radical choisi parmi H, $NO_2$, CN, NCO, COOH, $CONH_2$, alkyl, alkoxy, phényle, phénoxy.

La fixation de l'halogène et du groupe nitro sur le noyau se fera selon les règles de substitution bien connues de l'homme de l'art, en fonction de la présence de radicaux ortho, para ou méta orienteurs.

La fluoration échange permet l'échange des halogènes des groupes halogénométhyles, halogénométhoxyles et halogénothiométhyles en $-CF_3$, $-OCF_3$ et $-SCF_3$.

Dans le cas des groupes halogénoalkoxyles et halogénothioalkyles, l'échange se fera sur le carbone situé en α de l'hétéroatome. Ainsi, les groupes $-OCCl_2-CCl_3$, $-SCCl_2CCl_3$, seront transformés après fluoration-échange en $-OCF_2CCl_3$, $-SCF_2CCl_3$.

Par contre les atomes d'halogène directement fixés sur le noyau benzénique ne sont pas touchés par la fluoration-échange.

L'acide fluorhydrique utilisé pour la présente invention est de préférence de l'acide fluorhydrique anhydre.

Le rapport molaire de l'acide fluorhydrique au compose aromatique de départ est de préférence compris entre 10 et 100. Une quantité nettement supérieure n'est pas nuisible à l'invention.

La quantité d'halogène mise en oeuvre est fixée par l'homme de l'art compte-tenu du produit recherché correspondant à une mono ou polyhalogénation. Pour une monohalogénation, on opère de préférence en présence d'un défaut stoechiométrique d'halogène c'est-à-dire avec un rapport molaire halogène au composé aromatique compris de préférence entre 0,5 et 0,9. Pour une polyhalogénation, on préfère opérer avec un excès d'halogène. L'halogène peut être mis en oeuvre dans une enceinte fermée sous pression autogène (généralement 1 à 50 bars) ou sous pression atmosphérique par exemple par barbotage ou dans tout autre dispositif connu de l'homme de l'art.

L'agent nitrant est choisi parmi l'acide nitrique, les sels alcalins de l'acide nitrique, les sels de nitronium tel que le tétrafluoroborate de nitronium, l'hexafluorophosphate de nitronium, le trifluorométhane sulfonate de nitronium.

On utilise de préférence l'acide nitrique sous forme concentrée ou fumante.

L'acide nitrique peut aussi être généré in situ par action de l'acide fluorhydrique sur un de ses sels.

Dans le cas où l'on procède à la nitration préalablement à l'halogénation, pour minimiser les phénomènes d'hydrolyse, il est préférable de mettre en oeuvre un sel de nitronium et de préférence le tétrafluoroborate de nitronium.

Le rapport molaire de l'agent nitrant au composé aromatique de départ est de préférence au moins égal à 0,8 et encore plus préférentiellement compris entre 0,8 et 2.

Lorsque l'on procède à la nitration préalablement à l'halogénation et que l'on désire une mononitration, il est préférable que le rapport molaire sel de nitronium au composé aromatique soit d'environ 1.

La température de mise en oeuvre pour l'halogénation et la nitration est de préférence comprise entre -20° et 150°C.

La réaction peut avoir lieu à pression atmosphérique ou sous pression.

Lorsque la température sera supérieure à 20°C, la réaction devra avoir lieu sous pression puisque l'acide fluorhydrique doit être liquide.

Dans le cas où on réalise d'abord l'halogénation puis la nitration, une manière pratique de mettre en oeuvre l'invention est, à la fin de la réaction d'halogénation, d'arrêter l'alimentation en halogène, de laisser échapper l'halogène n'ayant pas réagi et éventuellement l'acide correspondant formé et d'introduire dans le milieu de réaction, sans aucune autre manipulation, l'acide nitrique.

La durée des réactions de chloration et de nitration varie de quelques minutes à quelques heures.

Le produit final qui est un dérivé aromatique halogéné, nitré et substitué par un groupe fluoroalkyle peut être extrait du milieu réactionnel par un solvant organique, puis lavé à l'eau plusieurs fois afin d'éliminer tout l'acide fluorhydrique et tout l'acide nitrique restant.

On peut selon un mode préférentiel d'extraction, distiller tout l'acide fluorhydrique puis éliminer l'acide nitrique par lavage à l'eau, du dérivé aromatique.

On peut citer comme produits de formule (I): le trichlorométhylbenzène, le trichlorométhoxybenzène, le trichlorométhylthiobenzène, les chlorotrichlorométhylbenzènes, les fluorotrichlorométhylbenzènes, le dichlorobromométhylbenzène, le tribromométhylbenzène, les chlorotrichlorométhoxybenzènes, les fluorotrichlorométhoxybenzènes, le chloroformiate de p-trichlorométhylphényle, le p-trichlorométhilphénylisocyanate, le pentachloroéthoxybenzène, le pentachloroéthylthiobenzène.

Bien que l'invention ne soit pas limitée à ces composés, elle trouve une application particulièrement intéressante pour la chloration, la nitration et la fluoration-échange des dérivés aromatiques perchloroalkyles, perchloroalkoxyles, et perchlorothioalkyles comme par exemple les trichlorométhylbenzènes, les trichlorométhoxybenzènes et les trichlorométhylthiobenzènes.

Un produit particulièrement intéressant du point de vue industriel est le trichlorométhylbenzène car, à partir du produit nitré et chloré, on peut préparer l'orthotrifluorométhylaniline utilisé comme intermédiaire de

synthèse dans la préparation de composés à activité phytosanitaire.

Les dérivés chlorés et nitrés aromatiques substitués par un groupe fluoroalkylés sont utilisés comme intermédiaires de synthèse dans l'industrie pharmaceutique, phytosanitaire et des colorants.

La présente invention sera plus aisément comprise à l'aide des exemples suivants donnés à titre indicatif mais nullement limitatif.

**Exemple 1**

Dans un réacteur de 250 ml, agité par un barreau aimanté et refroidi aux environs de 0° C, on introduit 100 ml (5 m) d'acide fluorhydrique anhydre et 19,6 g (0,1 m) de trichlorométhylbenzène. On laisse le mélange réactionnel se dégazer sous agitation (départ d'acide chlorhydrique) pendant une heure puis le réacteur est fermé et porté à la pression de 5 bars à 20° C par du chlore gazeux. On chauffe alors le tout, sous agitation à 100° C pendant 10 heures.

Après refroidissement de nouveau vers 0° C, le réacteur est décomprimé et on introduit goutte à goutte 6,9 g (0,11 $m^3$) d'acide nitrique 100 %. On laisse réagir 4 heures 30 mn à 20° C. Le brut réactionnel alors obtenu est coulé sur 120 g de glace pilée. Le mélange hétérogène issu de ce traitement est extrait par 3 fois 100 $cm^3$ de chlorure de méthylène. Après décantation, les phases organiques sont rassemblées. L'ensemble est lavé par 2 fois 100 $cm^3$ d'eau permutée et séché. Les analyses effectuées par chromatographie en phase gazeuse (% aire), spectrométrie IR et spectrométrie de masse nous donnent le résultat suivant:

| | |
|---|---|
| - m-nitrotrifluorométhylbenzène: | 12,3 % |
| - nitro-2 chloro-5 trifluorométhylbenzène: | 63,7 % |
| - autres nitrochlorotrifluorométhylbenzène: | 24 % |

**Exemple 2**

On procède de manière identique à l'exemple 1 avec les composés et conditions suivants:

| | |
|---|---|
| - Acide fluorhydrique anhydre: | 50 g (2,5 m) |
| - p-trichlorométhylphénylisocyanate: | 23,6 g (0,1 m) |
| - pression de chlore: | 4 bars à 20° C |
| - Température de chloration: | 20° C |
| - Temps de chloration: | 6 heures |
| - $HNO_3$ 100 %: | 7 g (0,11 eqm) |
| - Température de nitration: | 20° C |
| - Temps de nitration: | 4 heures |

Les analyses effectuées par chromatographie en phase gazeuse (% aire), spectrométrie IR et spectrométrie de masse nous donnent le résultat suivant:

- Fluorure de carbamoyle des trifluorométhyl-4 nitro-5 chloro-2 aniline et trifluorométhyl-4 nitro-6 chloro-2 aniline : 73 %

**Exemple 3**

On procède de manière identique à l'exemple 1 avec les composés et conditions ci-dessous et en remplaçant le traitement du brut réactionnel à la glace par une extraction de ce brut à l'aide de 2 fois 100 $cm^3$ de tétrachlorure de carbone, le traitement de ces phases organiques étant poursuivi normalement.

- Acide fluorhydrique anhydre: 100 g (5 m)
- Trichlorométhoxybenzène: 21,2 g (0,1 m)
- Brome: 16 g (0,1 m)
- Température de bromation: 120° C
- Temps de bromation: 5 heures
- $HNO_3$ 66%: 10,5 g (0,11 eqm)
- Température de nitration: 50° C
- Temps de nitration: 4 heures

Les analyses effectuées par chromatographie en phase gazeuse (% aire), spectrométrie IR et spectrométrie de masse nous donnent le résultat suivant:

- p-nitrotrifluorométhoxybenzène: 7 %
- nitro-2 bromo-4 trifluorométhoxybenzène et nitro-3 bromo-4 trifluorométhoxybenzène: 48 %

**Exemple 4**

On procède de manière identique à l'exemple 1 avec les composés et conditions ci-dessous et en remplaçant le traitement du brut réactionnel à la glace par une distillation jusqu'à 80° C en pied sous pression atmosphérique de ce brut afin d'éliminer au maximum l'acide fluorhydrique solvant.

- Acide fluorhydrique anhydre: 100 g (5 m)
- p-chlorotrichlorométhylbenzène: 23 g (0,1 m)
- Pression de chlore: 4 bars à 20° C
- Température de chloration: 100° C
- Temps de chloration: 4 heures
- $HNO_3$ 100 %: 6,3 g (0,1 m)
- Température de nitration: 30° C
- Temps de nitration: 3 heures

Les analyses effectuées par chromatographie en phase gazeuse (% aire), spectrométrie IR et spectrométrie de masse nous donnent le résultat suivant:

- nitro-3 chloro-4 trifluorométhylbenzène: 18 %
- nitro-2 dichloro-4,5 trifluorométhylbenzène et nitro-3 dichloro-4,5 trifluorométhylbenzène: 56 %

**Revendications**

1. Procédé d'halogénation-nitration-fluoration de dérivés aromatiques substitués par au moins un groupe comportant un motif halogénoalkyle caractérisé en ce que dans une même enceinte réactionnelle, on fait réagir sans traitement intermédiaire simultanément ou successivement le composé aromatique avec un halogène et un agent nitrant dans de l'acide fluorhydrique liquide.

2. Procédé selon la revendication 1 caractérisé en ce que on procède d'abord à l'halogénation puis à la nitration.

3. Procédé selon l'une quelconque des revendications 1 ou 2 caractérisé en ce que l'halogène est le chlore ou le brome.

4. Procédé selon la revendication 1 caractérisé en ce que le dérivé aromatique substitué par au moins un groupe comportant un motif halogénoalkyle répond à la formule générale suivante:

$Ar\text{-}(A\ CX_1X_2Y)_n$

dans laquelle:

- Ar représente un radical aromatique mono ou polycyclique

- A correspond à une liaison covalente, à l'oxygène ou au soufre;
- $X_1$ et $X_2$ identiques ou différents représentent un halogène
- Y correspond à l'un des groupes suivants: l'hydrogène, un halogène, une chaine alkyle de 1 à 3 atomes de carbone éventuellement halogénée. Les halogènes correspondant à $X_1$, $X_2$ et Y étant identiques ou différents, mais au moins l'un d'entre eux n'étant pas du fluor.
- n est égal à 1 ou 2.

5. Procédé selon la revendication 4 caractérisé en ce que n est égal à 1.

6. Procédé selon l'une des revendications 4 et 5 caractérisé en ce que Ar répond à la formule:

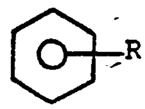

dans laquelle:
R représente un radical choisi parmi H, $NO_2$, CN, NCO, COOH, $CONH_2$, alkyl, alkoxy, phényle, phénoxy.

7. Procédé selon la revendication 1 caractérisé en ce que l'acide fluorhydrique est anhydre.

8. Procédé selon la revendication 1 caractérisé en ce que l'acide fluorhydrique est utilisé selon un rapport molaire acide fluorhydrique au dérivé aromatique compris entre 10 et 100.

9. Procédé selon la revendication 1, caractérisé en ce que l'agent nitrant est choisi parmi l'acide nitrique, les sels alcalins de l'acide nitrique, les sels de nitronium.

10. Procédé selon les revendications 1 et 9, caractérisé en ce que l'agent nitrant est utilisé selon un rapport molaire agent nitrant au dérivé aromatique d'au moins 0,8.

11. Procédé selon la revendication 10 caractérisé en ce que l'agent nitrant est utilisé selon un rapport molaire agent nitrant au dérivé aromatique compris entre 0,8 et 2.

12. Procédé selon l'une quelconque des revendications précédentes caractérisé en ce que l'halogénation, la nitration et la fluoration s'effectuent entre -20° C et 150° C.

13. Procédé selon la revendication 1 ou la revendication 4, caractérisé en ce que les dérivés aromatiques sont choisis parmi les dérivés perchloroalkylés, perchloroalkoxylés, perchlorothioalkylés.

14. Procédé selon la revendication 13, caractérisé en ce que les dérivés aromatiques sont choisis parmi les trichlorométhylbenzènes, trichlorométhoxybenzènes, trichlorométhylbenzènes.

## Patentansprüche

1. Verfahren zur Halogenierung-Nitrierung-Fluorierung aromatischer Verbindungen, die durch mindestens eine Gruppe, die eine Halogenoalkylgruppierung aufweist, substituiert sind, dadurch gekennzeichnet, daß man in einem einzigen Reaktionsbehälter ohne zwischenzeitliche Behandlung die aromatische Verbindung gleichzeitig oder aufeinanderfolgend mit einem Halogen und einem nitrierenden Agens in flüssiger Fluorwasserstoffsäure reagieren läßt.

2. Verfahren nach Anspruch 1, dadurch gekennzeichnet, daß man zunächst die Halogenierung und dann die Nitrierung durchführt.

3. Verfahren nach einem der Ansprüche 1 oder 2, dadurch gekennzeichnet, daß das Halogen, Chlor oder Brom ist.

4. Verfahren nach Anspruch 1, dadurch gekennzeichnet, daß die aromatische Verbindung durch mindestens eine Gruppe substituiert ist, die eine Halogenualkylbaueinheit aufweist entsprechend der nachfolgenden allgemeinen Formel:

$Ar\text{-}(A\ CX_1X_2Y)_n$

in der:
- Ar einen mono- oder polycyclischen aromatischen Rest darstellt
- A einer kovalenten Bindung zu Sauerstoff oder Schwefel entspricht;
- $X_1$ und $X_2$ identisch oder unterschiedlich sind und ein Halogen darstellen
- Y eine der nachfolgenden Gruppen darstellt: Wasserstoff, ein Halogen, eine Alkylkette mit 1 bis 3 Kohlenstoffatomen, gegebenenfalls halogeniert. Die $X_1$, $X_2$ und Y entsprechenden Halogene identisch oder unterschiedlich sind, jedoch mindestens eines von ihnen nicht Fluor ist.
- n gleich 1 oder 2 ist.

5. Verfahren nach Anspruch 4, dadurch gekennzeichnet, daß n gleich 1 ist.

6. Verfahren nach einem der Ansprüche 4 und 5, dadurch gekennzeichnet, daß Ar der Formel:

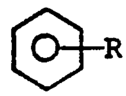

entspricht, in der:

R einen Rest ausgewählt aus H, $NO_2$, CN, NCO, COOH, $CONH_2$, Alkyl, Alkoxy, Phenyl, Phenoxy, darstellt.

7. Verfahren nach Anspruch 1, dadurch gekennzeichnet, daß die Fluorwasserstoffsäure wasserfrei ist.

8. Verfahren nach Anspruch 1, dadurch gekennzeichnet, daß die Fluorwasserstoffsäure entsprechend einem molaren Verhältnis Fluorwasserstoffsäure zur aromatischen Verbindung zwischen 10 und 100 eingesetzt wird.

9. Verfahren nach Anspruch 1, dadurch gekennzeichnet, daß das nitrierende Agens ausgewählt wird aus Salpetersäure, den Alkalisalzen der Salpetersäure und den Nitrylsalzen.

10. Verfahren nach den Ansprüchen 1 und 9, dadurch gekennzeichnet, daß das nitrierende Agens in einem molaren Verhältnis nitrierendes Agens zu aromatischer Verbindung von mindestens 0,8 eingesetzt wird.

11. Verfahren nach Anspruch 10, dadurch gekennzeichnet, daß das nitrierende Agens gemäß einem molarren Verhältnis nitrierendes Agens zu aromatischer Verbindung zwischen 0,8 und 2 eingesetzt wird.

12. Verfahren nach einem der vorhergehenden Ansprüche, dadurch gekennzeichnet, daß die Halogenierung, die Nitrierung und die Fluorierung bei zwischen -20° C und 150° C ausgeführt werden.

13. Verfahren nach Anspruch 1 oder Anspruch 4, dadurch gekennzeichnet, daß die aromatischen Verbindungen aus den perchloralkylierten, perchloralkoxylierten, perchlorthioalkylierten Verbindungen ausgewählt wurden.

14. Verfahren nach Anspruch 13, dadurch gekennzeichnet, daß die aromatischen Verbindungen aus den Trichlormethylbenzolen, Trichlormethoxybenzolen und Trichlormethylthiobenzolen ausgewählt wurden.

**Claims**

1. Process for the halogenation-nitration-fluorination of aromatic derivatives substituted by at least one group comprising a haloalkyl moiety, characterized in that the aromatic compound is reacted simultaneously or successively with a halogen and a nitrating agent in liquid hydrofluoric acid in the same single reaction enclosure without any intermediate treatment.

2. Process according to Claim 1, characterized in that the halogenation is first carried out and then the nitration.

3. Process according to either of Claims 1 and 2, characterized in that the halogen is chlorine or bromine.

4. Process according to Claim 1, characterized in that the aromatic derivative substituted by at least one group comprising a haloalkyl moiety corresponds to the following general formula:

$Ar\text{-}(A\ CX_1X_2Y)_n$

in which:

- Ar denotes a mono- or polycyclic aromatic radical
- A corresponds to a covalent bond, to oxygen or to sulphur;
- $X_1$ and $X_2$, which are identical or different, denote a halogen
- Y corresponds to one of the following groups: hydrogen, a halogen, an optionally halogenated alkyl chain containing 1 to 3 carbon atoms, the halogens corresponding to $X_1$, $X_2$ and Y being identical or different, but at least one of them not being fluorine;
- n is equal to 1 or 2.

5. Process according to Claim 4, characterized in that n is equal to 1.

6. Process according to either of Claims 4 and 5, characterized in that Ar corresponds to the formula:

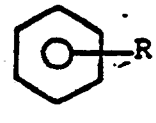

in which:

R denotes a radical chosen from H, $NO_2$, CN, NCO, COOH, $CONH_2$, alkyl, alkoxy, phenyl and phenoxy.

7. Process according to Claim 1, characterized in that the hydrofluoric acid is anhydrous.

8. Process according to Claim 1, characterized in that the hydrofluoric acid is employed in a molar ratio of hydrofluoric acid to the aromatic derivative of between 10 and 100.

9. Process according to Claim 1, characterized in that the nitrating agent is chosen from nitric acid, alkali metal salts of nitric acid, and nitronium salts.

10. Process according to Claims 1 and 9, characterized in that the nitrating agent is used in a molar ratio of nitrating agent to the aromatic derivative of at least 0.8.

11. Process according to Claim 10, characterized in that the nitrating agent is used in a molar ratio of nitrating

agent to the aromatic derivative of between 0.8 and 2.

12. Process according to any one of the preceding claims, characterized in that the halogenation, the nitration and the fluorination take place between -20° C and 150° C.

13. Process according to Claim 1 or Claim 4, characterized in that the aromatic derivatives are chosen from perchloroalkylated, perchloroalkoxylated and perchlorothioalkylated derivatives.

14. Process according to Claim 13, characterized in that the aromatic derivatives are chosen from trichloromethylbenzenes, trichloromethoxybenzenes and trichloromethylbenzenes.